# EUROPÄISCHE PATENTSCHRIFT

(11) **EP 2 039 294 B1**
(45) Veröffentlichungstag und Bekanntmachung des Hinweises auf die Patenterteilung: **18.05.2011**
(21) Anmeldenummer: 07018554.1
(22) Anmeldetag: 21.09.2007
(51) Int. Cl.: A61B 5/151

(54) **Stechsystem und Bandkassette**
Piercing system and tape cartridge
Système de connexion et cassette à bande magnétique

(43) Veröffentlichungstag der Anmeldung: 25.03.2009
(73) Patentinhaber: Roche Diagnostics GmbH, 68305 Mannheim (DE); F.Hoffmann-La Roche AG, 4070 Basel (CH)
(72) Erfinder: Harttig, Herbert, Dr., 67434 Neustadt (DE)
(74) Vertreter: Twelmeier, Ulrich

(56) Entgegenhaltungen:
- EP-A- 1 790 288
- WO-A-2005/107596
- DE-A1- 19 819 407

## Beschreibung

Die Erfindung betrifft ein Stechsystem mit den im Oberbegriff des Anspruchs 1 angegebenen Merkmalen und eine Bandkassette für ein Stechsystem. Ein derartiges Stechsystem ist aus der WO 2005/107596 A2 sowie aus der EP 1 790 288 A1 und der DE 198 19 407 A1 bekannt.

Ein solches Stechsystem ist ferner auch aus der nachveröffentlichten EP 1 967 139 A1 bekannt. Bei diesem Stechsystem ist das Trägerband zwischen einer ersten Wickelrolle und der Gebrauchsposition der Lanzetten um eine Vierteldrehung verdrillt. Auf dem Weg zu einer zweiten Wickelrolle ist das Trägerband um eine Vierteldrehung in entgegen gesetzter Richtung verdrillt. Die zweite Vierteldrehung macht so die erste Vierteldrehung wieder rückgängig.

Stechsysteme werden beispielsweise von Diabetikern benötigt, die mehrmals täglich ihren Blutzuckerspiegel überprüfen müssen und dafür eine aus einer Stichwunde gewonnene Körperflüssigkeitsprobe, in der Regel Blut oder interstitielle Flüssigkeit, benötigen. Stechsysteme können ein Stechgerät und austauschbare Bandkassetten mit Lanzettenträgerbändern umfassen oder als Wegwerfgeräte konzipiert sein, bei denen ein Austausch eines darin angeordneten Lanzettenträgerbandes nicht vorgesehen ist.

Mit einem Lanzettenträgerband, lässt sich ein großer Lanzettenvorrat platzsparend zur Verfügung stellen. Stechgeräte, die mit einem Lanzettenträgerband arbeiten, lassen sich deshalb trotz eines großen internen Lanzettenvorrats sehr kompakt ausbilden. Insbesondere für Benutzer, die ein Stechgerät ständig mit sich führen müssen, bedeutet dies eine große Entlastung.

Aufgabe der Erfindung ist es, einen Weg aufzuzeigen, Stechsysteme, die mit einem Lanzettenträgerband arbeiteten, weiter zu verbessern.

Diese Aufgabe wird erfindungsgemäß dadurch gelöst, dass Trägerband zwischen der ersten und der zweiten Wickelrolle nur in einer Drehrichtung um mindestens eine Vierteldrehung, vorzugsweise um mindestens eine Halbdrehung, verdrillt ist.

Durch eine Vierteldrehung des Trägerbandes lässt sich erreichen, dass sich ein Abschnitt des Trägerbandes quer zur geometrischen Drehachse der ersten Wickelrolle stellt. In diesem quer stehenden Abschnitt können Lanzetten zum Ausführen eines Stichs in einfacher Weise quer zur geometrischen Drehachse der ersten Wickelrolle bewegt werden. Stechgeräte können deshalb mit einer in einem Gehäuse liegenden Wickelrolle flach konzipiert werden und ergonomisch vorteilhaft in ein an eine Schmalseite des Geräts angelegtes Körperteil, in der Regel einen Finger, stechen.

Ob das Band nach dieser Vierteldrehung seine Orientierung beibehalten kann oder eine zusätzliche Verdrillung erforderlich ist, hängt insbesondere von der Orientierung der geometrischen Drehachse der zweiten Wickelrolle relativ zur Drehachse der ersten Wickelrolle ab. Bevorzugt sind die Drehachsen der beiden Wickelrollen parallel, so dass das Trägerband vor dem Aufwickeln auf die zweite Wickelrolle eine zweite Vierteldrehung ausführen sollte.

Prinzipiell könnte der Vorteil einer Vierteldrehung des Trägerbandes bei einem Gerät mit parallel verlaufenden geometrischen Drehachsen der Wickelrollen dadurch genutzt werden, dass die erste Vierteldrehung durch eine zweite Vierteldrehung mit entgegen gesetzter Drehrichtung wieder rückgängig gemacht wird. In einem solchen Fall wäre das Trägerband zwischen den beiden Wickelrollen in zwei unterschiedlichen Drehrichtungen verdrillt.

Wesentlich vorteilhafter ist es jedoch, wenn das Trägerband nur in einer einzigen Drehrichtung verdrillt ist. Bei einem Stechsystem mit parallel verlaufenden Drehachsen der Wickelrollen bedeutet dies, dass die erste Vierteldrehung nicht durch eine zweite Vierteldrehung rückgängig gemacht wird, sondern das Trägerband in der Drehrichtung der ersten Vierteldrehung weiter gedreht wird, vorzugsweise um genau eine weitere Vierteldrehung, so dass das Trägerband insgesamt um eine Halbdrehung verdrillt ist.

Wird das Trägerband nur einer Drehrichtung verdrillt, werden weniger Bandführungselemente benötigt als dies bei sich kompensierenden, entgegen gesetzten Vierteldrehung der Fall wäre. Weniger Bandführungselemente bedeuten nicht nur einen einfacheren und deshalb kostengünstigeren Aufbau des Stechsystems, sondern auch eine geringere Reibung.

Je weniger Bandführungselemente vorhanden sind, desto geringer ist nämlich in der Regel die Reibungsfläche, die auf das Trägerband einwirkt, bzw. der Umschlingungswinkel, um den das Trägerband durch Bandführungselemente umgelenkt wird. Eine reduzierte Reibung bedeutet, dass für den Bandtransport eine geringere Kraft ausreicht. Stechsysteme, bei denen der Bandtransport durch einen Elektromotor bewirkt wird, können deshalb mit einem leistungsschwächeren Motor verwirklicht werden und benötigen weniger elektrische Energie. Das für viele Benutzer lästige Wiederaufladen oder Austauschen von Batterien muss dann seltener durchgeführt werden oder das Gerät kann durch Verwendung kleinerer Batterien noch leichter und kompakter konzipiert werden. Bei einfacheren Stechsystemen, die einen Bandtransport durch eine von dem Benutzer aufzubringende Kraft vorsehen, bedeutet die reduzierte Reibung insbesondere für Benutzer, deren manuelle Beweglichkeit durch Alter oder Krankheit eingeschränkt ist, eine angenehme Entlastung.

Ein erfindungsgemäßes Stechsystem kann eine Bandkassette mit den im Anspruch 7 angegebenen Merkmalen und ein Stechgerät umfassen, in welches die Bandkassette eingesetzt wird und nach Gebrauch aller Lanzetten des Trägerbandes der Bandkassette ausgetauscht wird. Ein erfindungsgemäßes Stechsystem kann aber auch als ein Wegwerfgerät realisiert werden, bei dem ein Austausch des Trägerbandes nicht vorgesehen ist und das entsorgt wird, sobald alle Lanzetten des in dem Stechgerät angeordneten Trägerbandes benutzt wurden.

Weitere Einzelheiten und Vorteile der Erfindung werden anhand von Ausführungsbeispielen unter Bezugnahme auf die beigefügten Zeichnungen erläutert. Gleiche und einander entsprechende Teile sind dabei mit übereinstimmenden Bezugszahlen bezeichnet. Die im Rahmen der Ausführungsbeispiele beschriebenen Merkmale können einzeln und in Kombination zum Gegenstand von Ansprüchen gemacht werden.
Es zeigen:
- Figur 1:: ein Ausführungsbeispiel eines erfindungsgemäßen Stechsystems bei geöffnetem Gehäuse;
- Figur 2:: eine schematische Darstellung der Bandführung;
- Figur 3:: eine schematische Darstellung eines Ausführungsbeispiels einer erfindungsgemäßen Bandkassette;
- Figur 4:: ein Ausführungsbeispiel eines Stechgeräts zum Gebrauch der in Figur 3 gezeigten Bandkassette;
- Figur 5: eine schematische Darstellung der Bandaustrittsöffnung der in Figur 3 gezeigten Bandkassette; und
- Figur 6: eine schematische Darstellung der Bandeintrittsöffnung der in Figur 3 gezeigten Bandkassette.

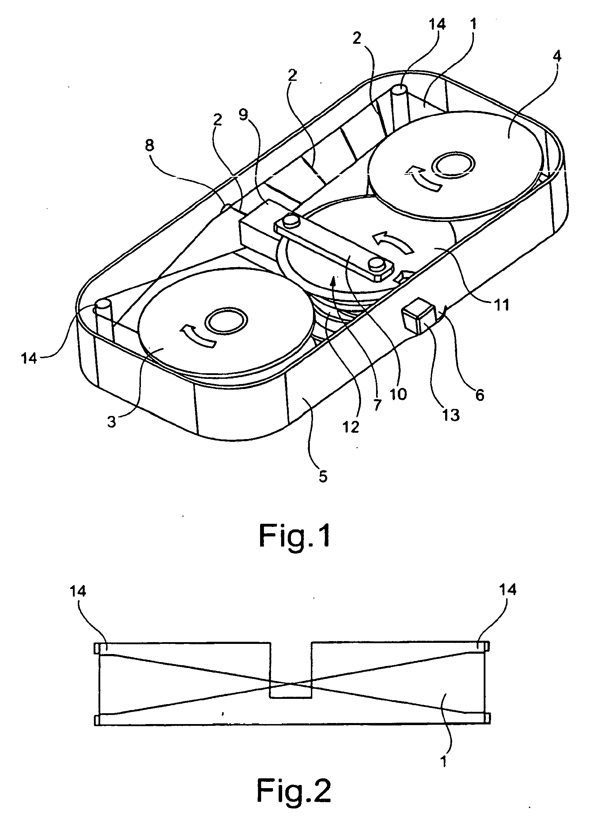

Figur 1 zeigt ein Ausführungsbeispiel eines Stechsystems bei geöffnetem Gerätegehäuse mit einem Trägerband 1, das mehrere quer zu seiner Längsrichtung orientierte Lanzetten 2 trägt. Das Trägerband 1 ist mit unbenutzten Lanzetten 2 auf eine erste Wickelrolle 3 aufgewickelt. Trägerbandabschnitte benutzter Lanzetten 2 werden auf eine zweite Wickelrolle 4 aufgewickelt. Die zweite Wickelrolle 4 wird von einem Wickelantrieb angetrieben, der bei dem dargestellten Ausführungsbeispiel als ein aus dem Gerätegehäuse 5 herausragendes Antriebsrad 6 ausgebildet ist. Durch Drehen der zweiten Wickelrolle 4 lassen sich die von dem Trägerband 1 getragenen Lanzetten 2 nacheinander in eine Gebrauchsposition bringen, wobei das Trägerband 1 von der ersten Wickelrolle 3 ab und auf die zweite Wickelrolle 4 aufgewickelt wird. In der Gebrauchsposition können die Lanzetten 2 mit einem Stechantrieb 7 für einen Stich beschleunigt werden, um in einem an eine Geräteöffnung 8 angelegten Körperteil eine Stichwunde zur Gewinnung einer Körperflüssigkeitsprobe zu erzeugen.

Der Lanzettenantrieb 7 umfasst einen Antriebskopf 9, der einen Schlitz aufweist, in dem das Trägerband 1 gehalten ist. Der Antriebskopf 9 wird über eine Pleuelstange 10 angetrieben, die mit einem Rotor 11 gekoppelt ist, der von einer Antriebsfeder 12, die bevorzugt als Spiralfeder ausgebildet ist, angetrieben wird. Die Antriebsfeder 12 lässt sich durch Betätigung des Antriebsrades 6 spannen, das zugleich dem Bandtransport dient. Ein Auslöseelement 13, bevorzugt eine Taste, dient zum Auslösen einer Stichbewegung.

Das Trägerband 1 ist zwischen den beiden Wickelrollen 3, 4 über zwei Bandführungselemente 14 geführt, zwischen denen sich die Gebrauchsposition befindet. Die Bandführungselemente 14 sind als Umlenkungen ausgeführt. Die Umlenkungen können als Stifte oder Gehäusekante ausgebildet sein. Bevorzugt handelt es sich bei den Umlenkungen um Rollen, die beispielsweise als drehbar auf Stiften gelagerte Hülsen ausgeführt sein können. Die Bandführungselemente 14 könnten beispielsweise auch als einfache Stifte ausgeführt sein. Rollen haben den Vorteil, einen Bandtransport mit weniger Reibung zu ermöglichen.

Eine Besonderheit des dargestellten Stechsystems besteht darin, dass das Trägerband 1 zwischen der ersten und der zweiten Wickelrolle nur in einer Drehrichtung verdrillt ist. Insgesamt ist das Trägerband 1 zwischen den beiden Wickelrollen 3, 4 um eine Halbdrehung verdrillt. Die Gebrauchsposition, in der eine Lanzette 2 für einen Stich in ein an die Gehäuseöffnung 8 angelegtes Körperteil benutzt werden kann, befindet sich in dem um die Halbdrehung verdrillten Bandabschnitt, wobei das Trägerband 1 auf beiden Seiten der Gebrauchsposition um jeweils eine Vierteldrehung verdrillt ist. Mit der ersten Vierteldrehung zwischen der ersten Wickelrolle 3 und der Gebrauchsposition wird das Trägerband 1 in eine Orientierung gebracht, in der die von dem Band 1 getragenen Lanzetten 2 quer, vorzugsweise senkrecht, zur geometrischen Drehachse der ersten Wickelrolle 3 stehen.

Bei dem dargestellten Ausführungsbeispiel sind die Lanzetten 2 quer zur Längsrichtung des Trägerbandes 1 angeordnet und folglich nach der ersten Vierteldrehung in Stichrichtung ausgerichtet.

Bei der zweiten Vierteldrehung, die denselben Drehsinn wie die erste Vierteldrehung hat, werden die Lanzetten 2 wieder aufgerichtet, so dass sie sich längs, vorzugsweise parallel, zur geometrischen Drehachse der zweiten Wickelrolle 4 erstrecken. Die geometrischen Drehachsen der beiden Wickelrollen 3, 4 sind bevorzugt parallel, können jedoch auch eine davon abweichende Orientierung haben.

Indem das Trägerband 1 nur in einer Drehrichtung verdrillt ist, sind neben den beiden vorstehend erwähnten Bandführungselementen 14 keine weiteren Bandführungselemente erforderlich. Insbesondere sind zum Erzeugen der Vierteldrehungen zwischen der Gebrauchsposition und den beiden Wickelrollen 3, 4 keine gesonderten Bandführungselemente erforderlich. Das dargestellte Stechsystem ermöglicht deshalb einen besonders reibungsarmen Bandtransport.

Die Verdrillung des Trägerbandes 1 zwischen den beiden Bandführungselementen 14 ist in Figur 2 schematisch mit Blickrichtung entgegen der Stichrichtung dargestellt.

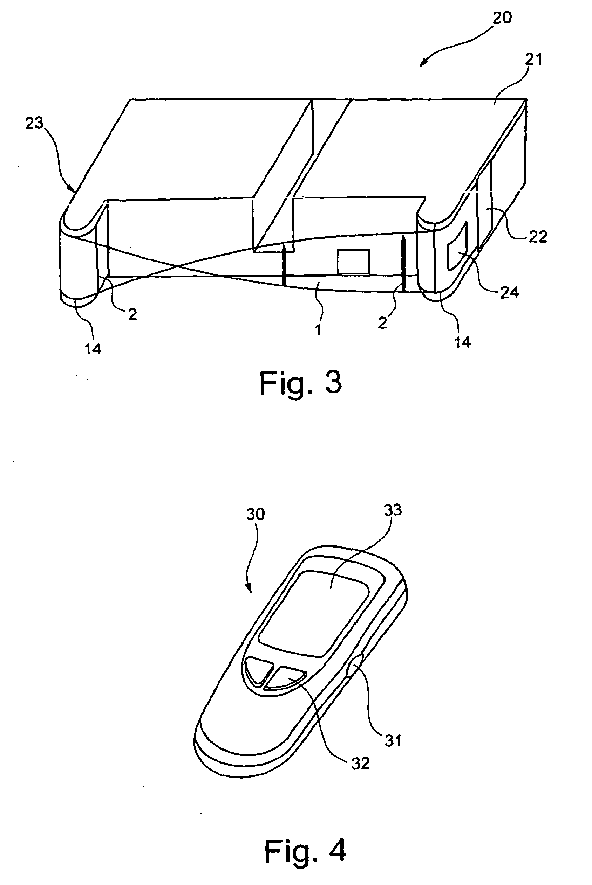

Figur 3 zeigt eine schematische Darstellung eines Ausführungsbeispiels einer Bandkassette 20 zum Gebrauch in einem Stechgerät 30, wie es beispielsweise in Figur 4 gezeigt ist.

Die Bandkassette 20 hat ein Gehäuse 21, in dem ein Trägerband 1 angeordnet ist, das mehrere Lanzetten 2 trägt, die bevorzugt quer zur Längsrichtung des Bandes 1 angeordnet sind. Das Trägerband 1 ist in der Bandkassette 20 ähnlich wie bei dem in Figur 1 dargestellten Ausführungsbeispiel auf eine erste Wickelrolle aufgewickelt, von der es sich durch Drehen einer zweiten Wickelrolle abwickeln und auf die zweite Wickelrolle aufwickeln lässt (in Figur nicht dargestellt). Ebenso wie bei dem in Figur 1 dargestellten Stechsystem ist das Trägerband 1 der in Figur 3 dargestellten Bandkassette 20 nur in einer Drehrichtung verdrillt, bevorzugt um eine Halbdrehung, verdrillt.

Das Trägerband 1 tritt aus einer Austrittsöffnung 22 aus dem Gehäuse 21 der Bandkassette 20 heraus und durch eine Eintrittsöffnung 23 wieder in das Gehäuse 21 ein. Das Trägerband 1 ist zwischen der Austrittsöffnung 22 und der Eintrittsöffnung 23 um eine Halbdrehung verdrillt.

Das Trägerband 1 des in Figur 3 dargestellten Ausführungsbeispiels trägt zusätzlich zu den Lanzetten 2 Testfelder 24 zur Untersuchung einer durch einen Lanzettenstich gewonnenen Körperflüssigkeitsprobe. Die Testfelder 24 weisen Nachweisreagenzien auf, die eine photometrische oder elektrochemische Bestimmung einer Analytkonzentration, beispielsweise der Glucosekonzentration, ermöglichen. Entsprechende Testfelder sind bei handelsüblichen Teststreifen, beispielsweise zur Blutzuckerbestimmung, vorhanden und bedürfen deshalb keiner weiteren Erläuterung. Bevorzugt sind die Testfelder 24 zwischen den Lanzetten 2 angeordnet.

Figur 4 zeigt ein Ausführungsbeispiel eines Stechgeräts 30, in das die Bandkassette 20 zum Gebrauch eingelegt werden kann. Das Stechgerät 30 hat ein Aufnahmefach (nicht dargestellt) für die Bandkassette 20. Das Aufnahmefach hat eine verschließbare Öffnung, die sich auf der Rückseite des in Figur 4 dargestellten Ausführungsbeispiels befindet.

Das Stechgerät 30 weist eine Geräteöffnung 31 auf, gegen die ein Körperteil zum Erzeugen einer Stichwunde gepresst wird. Das Stechgerät 30 hat ferner Bedienungselemente 32, beispielsweise Tasten, und eine Anzeigeeinrichtung 33, beispielsweise eine Flüssigkristallanzeige.

Das dargestellte Stechgerät 30 enthält einen Wickelantrieb, um die zweite Wickelrolle einer eingesetzten Bandkassette 20 zu drehen und dadurch die von dem Trägerband 1 getragenen Lanzetten 2 und Testfelder 24 nacheinander in eine Gebrauchsposition zu bringen. Der Wickelantrieb ist ebenso wie ein in dem Stechgerät 30 enthaltener Stechantrieb vorzugsweise batteriebetrieben. Der Stechantrieb kann im übrigen ebenso wie der Stechantrieb des in Figur 1 dargestellten Ausführungsbeispiels ausgebildet sein, wobei zum Spannen der Antriebsfeder ein Elektromotor dient.

Der Stechantrieb beschleunigt nicht nur Lanzetten 2, die sich in der Gebrauchsposition befinden, für einen Stich, sondern auch sich in der Gebrauchsposition befindende Testfelder 24 für eine Probenaufnahmebewegung in Stichrichtung.

Das dargestellte Stechgerät 30 enthält bevorzugt auch eine Messeinrichtung, um das Resultat einer mit einem Testfeld 24 und einer aufgenommenen Körperflüssigkeitsprobe durchgeführten Nachweisreaktion zu messen, so dass eine Analytkonzentration bestimmt werden kann.

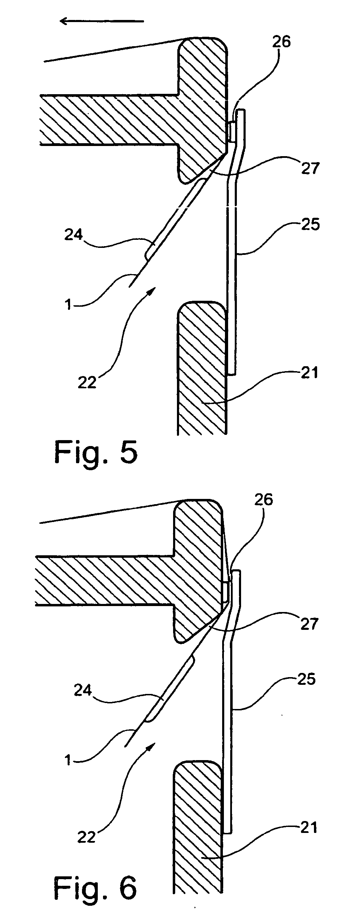

Testfelder mit Nachweisreagenzien, wie sie auf dem Trägerband des in Figur 3 gezeigten Ausführungsbeispiels vorhanden sind, sind in der Regel feuchtigkeitsempfindlich. Um die Testfelder 24 des Trägerbandes 1 vor Feuchtigkeit und anderen schädlichen Umwelteinflüssen zu schützen, können die Bandaustrittsöffnung 22 und die Bandeintrittsöffnung 23 der Bandkassette 20 jeweils mit einer Durchzugsdichtung versehen werden. Beispiele geeigneter Durchzugsdichtungen sind in den Figuren 5 und 6 gezeigt.

Wegen der Halbdrehung, um die das Trägerband 1 zwischen der Bandaustrittsöffnung 22 und der Bandeintrittsöffnung 23 verdrillt ist, sind die von dem Band getragenen Testfelder 24 bei einem Gehäusedurchtritt, beispielsweise beim Bandaustritt, der Bandkassette 20 zugewandt und bei dem anderen Gehäusedurchtritt, beispielsweise beim Bandeintritt, von der Bandkassette 20 abgewandt.

Bei dem beschriebenen Ausführungsbeispiel sind die Testfelder 24 beim Bandaustritt gemäß Figur 5 orientiert und beim Bandeintritt gemäß Figur 6 orientiert. Die Testfelder 24 sind also nach Austreten aus dem Gehäuse 21 der Bandkassette 20 zunächst der Bandkassette zugewandt und hinter der Halbdrehung von dem Gehäuse 21 abgewandt. Prinzipiell könnte es aber auch umgekehrt sein.

Die in Figur 5 gezeigte Durchzugsdichtung wird von einer die Austrittsöffnung 22 bedeckenden Folie 25 und einer daran befestigten Dichtungslippe 26 gebildet. Das Band liegt mit einer Bandseite an dem Gehäuse 21 und mit der anderen Bandseite an der Dichtungslippe 26 an. Um den Bandtransport zu erleichtern hat das Gehäuse 21 am Rand der Austrittsöffnung 22 eine Abschrägung 27. Die Testfelder 24 können mit geringem Reibungswiderstand über die Schrägfläche 27 des Gehäuses 21 gleiten.

Die Dichtungslippe 26 ist aus einem weichen Kunststoffmaterial, beispielsweise Schaumstoff, und drückt nur auf die glatte Bandseite, da sich die Testfelder 24 auf der gegenüberliegenden Bandseite befinden. Obwohl die Dichtungslippe 26 weich und komprimierbar ist bewirkt sie deshalb nur einen geringen Reibungswiderstand.

Bei der Bandeintrittsöffnung 23 ist die Dichtungslippe 26 dagegen an dem Gehäuse 21 befestigt, so dass das Band 1 zwischen Folie 25 und Dichtungslippe 26 hindurchgeführt wird. Auf diese Weise wird vermieden, dass sich die weiche Dichtungslippe 26 an von Testfeldern 24 und Lanzetten 2 gebildeten Unebenheiten verhakt.

Auf eine Durchzugsdichtung der Bandeintrittsöffnung 23 kann vorteilhafter Weise verzichtet werden, indem die erste Wickelrolle, um die unbenutzte Abschnitte des Trägerbandes 1 gewickelt sind, in einer gegenüber der Bandeintrittsöffnung 23 abgedichteten Kammer angeordnet ist, was bevorzugt ist. Die Bandkassette 20 enthält dann vorzugsweise zwei separate Kammern, in denen jeweils eine der beiden Wickelrollen angeordnet ist. Unbenutzte Testfelder 24 können in der Bandkassette 20 zusätzlich durch Trockenmittel geschützt werden.

### Bezugszahlen

- 1: Trägerband
- 2: Lanzetten
- 3: erste Wickelrolle
- 4: zweite Wickelrolle
- 5: Gerätegehäuse
- 6: Antriebsrad
- 7: Stechantrieb
- 8: Geräteöffnung
- 9: Antriebskopf
- 10: Pleuelstange
- 11: Rotor
- 12: Antriebsfeder
- 13: Auslöseelement
- 14: Bandführungselemente
- 20: Bandkassette
- 21: Gehäuse
- 22: Austrittsöffnung
- 23: Eintrittsöffnung
- 24: Testfelder
- 25: Folie
- 26: Dichtungslippe
- 27: Abschrägung
- 30: Stechgerät
- 31: Geräteöffnung
- 32: Bedienungselemente
- 33: Anzeigeeinrichtung

## Patentansprüche

1. Stechsystem mit
einem Trägerband (1), das mehrere Lanzetten (2) trägt,
einer ersten Wickelrolle (3), auf die das Trägerband (1) mit unbenutzten Lanzetten (2) aufgewickelt ist,
einer zweiten Wickelrolle (4), um Trägerbandabschnitte benutzter Lanzetten (2) aufzuwickeln,
einem Wickelantrieb (6), um durch Drehen der zweiten Wickelrolle (4) die von dem Trägerband (1) getragenen Lanzetten (2) nacheinander in eine Gebrauchsposition zu bringen und dabei das Trägerband (1) von der ersten Wickelrolle (3) ab und auf die zweite Wickelrolle (4) aufzuwickeln,
einem Stechantrieb (7), um Lanzetten (2), die sich in der Gebrauchsposition befinden, für einen Stich zu beschleunigen,
**dadurch gekennzeichnet, dass** das Trägerband (1) zwischen der ersten und der zweiten Wickelrolle (3, 4) nur in einer Drehrichtung um mindestens eine Vierteldrehung, vorzugsweise um mindestens eine Halbdrehung, verdrillt ist.

2. Stechsystem nach Anspruch 1, **dadurch gekennzeichnet, dass** sich die Gebrauchsposition in dem um die Halbdrehung verdrillten Bandabschnitt befindet, so dass das Trägerband (1) auf beiden Seiten der Gebrauchsposition um jeweils eine Vierteldrehung verdrillt ist.

3. Stechsystem nach einem der vorstehenden Ansprüche, **dadurch gekennzeichnet, dass** das Trägerband (1) zwischen den beiden Wickelrollen (3, 4) über mindestens eine, vorzugsweise zwei, Umlenkungen (14) geführt ist.

4. Stechsystem nach Anspruch 3, **dadurch gekennzeichnet, dass** das Trägerband (1) zwischen zwei Umlenkungen (14) um die Halbdrehung verdrillt ist.

5. Stechsystem nach einem der vorstehenden Ansprüche, **dadurch gekennzeichnet, dass** der Wickelantrieb (6) und der Stechantrieb (7) Teil eines Stechgeräts (30) sind, in das eine Bandkassette (20), die das Trägerband (1) und die beiden Wickelrollen (3, 4) enthält, einsetzbar ist.

6. Stechsystem nach einem der vorstehenden Ansprüche, **dadurch gekennzeichnet, dass** die Lanzetten (2) quer zur Längsrichtung des Trägerbandes (1) angeordnet sind.

7. Bandkassette zum Gebrauch in einem Stechgerät (30), mit
einem Trägerband (1), das mehrere Lanzetten (2) trägt,
einer ersten Wickelrolle (3), auf die das Trägerband (1) aufgewickelt ist, und
einer zweiten Wickelrolle (4), wobei sich das Trägerband (1) durch Drehen der zweiten Wickelrolle (4) von der ersten Wickelrolle (3) abwickeln und auf die zweite Wickelrolle (4) aufwickeln lässt, **dadurch gekennzeichnet, dass** das Trägerband (1) zwischen der ersten und der zweiten Wickelrolle (3, 4) nur in einer Drehrichtung um mindestens eine Vierteldrehung, vorzugsweise um mindestens eine Halbdrehung, verdrillt ist.

8. Bandkassette nach Anspruch 7, **dadurch gekennzeichnet, dass** das Trägerband (1) zusätzlich zu den Lanzetten (2) auch Testfelder (24) zum Untersuchen von Körperflüssigkeitsproben trägt.

9. Bandkassette nach Anspruch 7 oder 8, **gekennzeichnet durch** eine Austrittsöffnung (22), **durch** die das Trägerband (1) aus einem Gehäuse (21) der Bandkassette (20) heraustritt, und eine Eintrittsöffnung (23), **durch** die das Trägerband (1) wieder in das Gehäuse (21) der Bandkassette (20) eintritt, wobei das Trägerband (1) zwischen der Austrittsöffnung (22) und der Eintrittsöffnung (23) um eine Halbdrehung verdrillt ist.

10. Bandkassette nach Anspruch 9, **dadurch gekennzeichnet, dass** die Austrittsöffnung (22) mit einer Durchzugsdichtung versehen ist, bei der das Trägerband (1) zwischen dem Gehäuse (21) der Bandkassette (20) und einer Dichtungslippe (26), die an einer die Austrittsöffnung (22) bedeckenden Folie (25) befestigt ist, hindurchgeführt ist.

## Claims

1. Puncturing system having
a carrier tape (1) carrying a plurality of lancets (2),
a first reel (3) on which the carrier tape (1) with unused lancets (2) is wound up,
a second reel (4) for winding up carrier tape portions with used lancets (2),
a reel drive (6) intended to rotate the second reel (4) and to thereby bring the lancets (2) carried by the carrier tape (1) successively into a position for use, and thereby to unwind the carrier tape (1) from the first reel (3) and to wind it up on the second reel (4),
a puncturing drive (7) for accelerating lancets (2), that are in the position for use, for a puncturing action,
**characterized in that** the carrier tape (1) is twisted between the first and the second reels (3, 4) only in one direction by at least one quarter turn, preferably by at least one half turn.

2. The puncturing system as defined in Claim 1, **characterized in that** the position for use is located in the tape portion that has been twisted by a half turn so that the carrier tape (1) is twisted by a quarter turn on each side of the position for use.

3. The puncturing system as defined in any of the preceding claims, **characterized in that** the carrier tape (1) is guided between the two reels (3, 4) over at least one, preferably two tape guiding elements (14).

4. The puncturing system as defined in Claim 3, **characterized in that** the carrier tape (1) is twisted by the half turn between two tape guiding elements (14).

5. The puncturing system as defined in any of the preceding claims, **characterized in that** the reel drive (6) and the puncturing drive (7) are part of a puncturing device (30) into which can be loaded a tape cassette (20), which contains the carrier tape (20) and the two reels (3, 4).

6. The puncturing system as defined in any of the preceding claims, **characterized in that** the lancets (2) are arranged transversely to the longitudinal direction of the carrier tape (1).

7. Tape cassette for use in a puncturing device (30), comprising
a carrier tape (1) that carries a plurality of lancets (2),
a first reel (3) on which the carrier tape (1) is wound up, and
a second reel (4), where the carrier tape (1) can be unwound from the first reel (3) and can be wound up on the second reel (4) by rotation of the second reel (4), **characterized in that** the carrier tape (1) is twisted between the first and the second reels (3, 4) only in one direction by at least one quarter turn, preferably by at least one half turn.

8. The tape cassette as defined in Claim 7, **characterized in that** the carrier tape (1) carries test fields (24) for examination of samples of a body liquid, in addition to the lancets (2).

9. The tape cassette as defined in Claim 7 or Claim 8, **characterized by** an exit opening (22), through which the carrier tape (1) exits from a housing (21) of the tape cassette (20), and an entry opening (23) through which the carrier tape (1) enters again the housing (21) of the carrier tape (20), the carrier tape (1) being twisted by one half turn between the exit opening (22) and the entry opening (23).

10. The carrier tape as defined in Claim 9 **characterized in that** the exit opening (22) is provided with a passage seal where the carrier tape (1) passes between the housing (21) of the tape cassette (20) and a sealing lip (26) fastened to a film (25) that covers the exit opening (22).

## Revendications

1. Système de piqûre comprenant
un ruban porteur (1) qui porte plusieurs lancettes (2),
une première bobine d'enroulement (3) sur laquelle le ruban porteur (1) avec les lancettes non utilisées (2) est enroulé,
une seconde bobine d'enroulement (4) pour enrouler des portions de ruban porteur avec des lancettes utilisées (2),
un mécanisme d'enroulement (6) pour amener les lancettes (2) portées par le ruban porteur (1) les unes après les autres dans une position d'utilisation par rotation de la seconde bobine d'enroulement (4), et pour dérouler ainsi le ruban porteur (1) de la première bobine d'enroulement (3) et l'enrouler sur la seconde bobine d'enroulement (4),
une commande de piqûre (7) pour accélérer les lancettes (2) qui se trouvent dans la position d'utilisation pour réaliser une piqûre,
**caractérisé en ce que** le ruban porteur (1) est torsadé d'au moins un quart de tour, de préférence d'au moins un demi-tour, dans un seul sens de torsion, entre la première et la seconde bobines d'enroulement (3, 4).

2. Système de piqûre selon la revendication 1, **caractérisé en ce que** la position d'utilisation se trouve dans la portion de ruban torsadé d'un demi-tour, de sorte que le ruban porteur (1) est torsadé à chaque fois d'un quart de tour sur les deux côtés de la position d'utilisation.

3. Système de piqûre selon l'une des revendications précédentes, **caractérisé en ce que** le ruban porteur (1) est guidé sur au moins un, de préférence deux éléments de déviation (14), entre les deux bobines d'enroulement (3, 4).

4. Système de piqûre selon la revendication 3, **caractérisé en ce que** le ruban porteur (1) est torsadé d'un demi-tour entre deux éléments de déviation (14).

5. Système de piqûre selon l'une des revendications précédentes, **caractérisé en ce que** le mécanisme d'enroulement (6) et la commande de piqûre (7) font partie d'un dispositif de piqûre (30) dans lequel peut être insérée une cassette à ruban (20) qui contient le ruban porteur (1) et les deux bobines d'enroulement (3, 4).

6. Système de piqûre selon l'une des revendications précédentes, **caractérisé en ce que** les lancettes (2) sont placées perpendiculairement à la direction longitudinale du ruban porteur (1).

7. Cassette à ruban destinée à être utilisé dans un dispositif de piqûre (30) comprenant
un ruban porteur (1) qui porte plusieurs lancettes (2),
une première bobine d'enroulement (3) sur laquelle le ruban porteur (1) est enroulé et
une seconde bobine d'enroulement (4), le ruban porteur (1) pouvant être déroulé de la première bobine d'enroulement (3) et enroulé sur la seconde bobine d'enroulement (4) par rotation de la seconde bobine d'enroulement (4), **caractérisée en ce que** le ruban porteur (1) est torsadé d'au moins un quart de tour, de préférence d'au moins un demi-tour, dans un seul sens de torsion, entre la première et la seconde bobines d'enroulement (3, 4).

8. Cassette à ruban selon la revendication 7, **caractérisée en ce que** le ruban porteur (1) porte également, en plus des lancettes (2), des champs de test (24) pour l'analyse d'échantillons de fluides corporels.

9. Cassette à ruban selon la revendication 7 ou 8, **caractérisée par** une ouverture de sortie (22) à travers laquelle le ruban porteur (1) sort d'un boîtier (21) de la cassette à ruban (20) et une ouverture d'entrée (23) à travers laquelle le ruban porteur (1) entre à nouveau dans le boîtier (21) de la cassette à ruban (20), le ruban porteur (1) étant torsadé d'un demi-tour entre l'ouverture de sortie (22) et l'ouverture d'entrée (23).

10. Cassette à ruban selon la revendication 9, **caractérisé en ce que** l'ouverture de sortie (22) est pourvue d'une traversée étanche au niveau de laquelle le ruban porteur (1) est amené à passer entre le boîtier (21) de la cassette à ruban (20) et une lèvre d'étanchéité (26) qui est fixée sur une feuille (25) couvrant l'ouverture de sortie (22).
